# EUROPEAN PATENT APPLICATION

(11) **EP 4 306 124 A1**
(43) Date of publication of application: **17.01.2024**
(21) Application number: 22767554.3
(22) Date of filing: 11.03.2022
(51) Int. Cl.: A61K 38/51, A61K 38/20, A61K 38/18, A61K 9/00, A61P 25/28

(54) **COMPOSITION FOR PREVENTING OR TREATING DEGENERATIVE BRAIN DISEASES**

(30) Priority: 12.03.2021 KR 20210032759
(71) Applicant: Kolon Life Science, Inc., Seoul 07793 (KR)
(72) Inventor: PARK, Jangjoon, Seoul 07793 (KR); SHIN, Jaeil, Seoul 04731 (KR); HONG, Soonoh, Seoul 07692 (KR); KIM, Minju, Seoul 03382 (KR); KIM, Kyungran, Yongin-si, Gyeonggi-do 16874 (KR); JI, Hyelin, Seoul 06374 (KR); SIM, Yeomoon, Seoul 05266 (KR); KIM, Haeun, Seoul 07593 (KR); LEE, Juyoun, Seoul 08026 (KR); LEE, Hyejin, Incheon 21558 (KR)
(74) Representative: Ostertag & Partner Patentanwälte mbB
(86) International application number: PCT/KR2022/003444
(87) International publication number: WO 2022/191658

(57) **Abstract**

The present invention relates to a composition for preventing or treating degenerative brain diseases. The pharmaceutical composition of the present invention contains at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same. Since the pharmaceutical composition of the present invention exhibits a better effect of alleviating and preventing degenerative brain diseases by co-administration of active ingredients than upon single administration, the pharmaceutical composition of the present invention can exhibit the same or higher effect only with a low concentration of the active ingredients. Therefore, the pharmaceutical composition of the present invention can be advantageously used for the prevention or treatment of degenerative brain diseases.

## Description

### [Technical Field]

The present invention relates to a composition for preventing or treating degenerative brain diseases and a method for preventing or treating degenerative brain diseases using the same.

### [Background Art]

A degenerative brain disease is currently one of the most socially problematic diseases due to an increase in the elderly population, and is a disease whose seriousness is becoming more and more evident because no specific therapeutic agent has been developed yet, even though there is growing interest in its prevention and treatment. A neurodegenerative disease is a general term for diseases caused by the death of neurons, which is progressing slowly and steadily in one or more parts of the nervous system, and a degenerative brain disease may occur when the brain is damaged by the structural deterioration of brain neurons due to aging, secondary symptoms caused by adult diseases such as circulatory disorders, or physical and mechanical factors such as traffic accidents, industrial accidents, and carbon monoxide poisoning, or even when brain tissue is irreversibly damaged by reduced or blocked blood flow to the brain, but the exact cause is currently unknown. When a degenerative brain disease occurs, progressive cognitive dysfunction, progressive ataxia, muscle weakness, amyotrophy, and the like may occur depending on the site and pathogenesis of the disease, and the like.

Each of various genes is known to be effective in suppressing neuronal cell death. However, there is a limit to how much a single gene can suppress the death of neurons, and single gene therapy is not free from side effects such as toxicity because there is no choice but to use high-concentration gene therapeutic agents. Therefore, there is need for discovering a therapeutic agent capable of not only effectively preventing or treating degenerative brain diseases without side effects, but also exhibiting a synergistic effect on the preventive or therapeutic effects of degenerative brain diseases according to the co-administration of gene therapeutic agents.

### [Disclosure]

### [Technical Problem]

Under the background as described above, the present inventors conducted intensive studies to develop a therapeutic agent having preventive or therapeutic effects against degenerative brain diseases, and as a result, confirmed that when at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same are used, the case shows a remarkably excellent preventive or therapeutic effect on degenerative brain diseases compared to a use of only one, thereby completing the present invention.

Thus, an object of the present invention is to provide a pharmaceutical composition for preventing or treating a degenerative brain disease, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

Further, another object of the present invention is to provide a health functional food for preventing or ameliorating a degenerative brain disease, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

In addition, still another object of the present invention is to provide a method for preventing or treating a degenerative brain disease, the method including administering at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same to an individual in need thereof.

Furthermore, yet another object of the present invention is to provide a use of at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same for preparing a preventive or therapeutic agent for degenerative brain diseases.

However, technical problems to be solved by the present invention are not limited to the aforementioned problems, and other problems that are not mentioned may be clearly understood by those skilled in the art from the following description.

### [Technical Solution]

To achieve the objects, the present invention provides a pharmaceutical composition for preventing or treating a degenerative brain disease, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

As an exemplary embodiment of the present invention, the pharmaceutical composition may contain the IL-10 protein or the gene encoding the same and the GDNF protein or the gene encoding the same.

As another exemplary embodiment of the present invention, the pharmaceutical composition may further contain the GAD protein or the gene encoding the same.

As still another exemplary embodiment of the present invention, the gene may be contained in a vector.

As yet another exemplary embodiment of the present invention, the vector may be a viral or non-viral vector.

As yet another exemplary embodiment of the present invention, the viral vector may be at least one selected from the group consisting of adenoviruses, adeno-associated viruses (AAV), herpes simplex virus, lentiviruses, retroviruses, cytomegalovirus, baculoviruses, and poxviruses.

As yet another exemplary embodiment of the present invention, the non-viral vector may be at least one selected from the group consisting of plasmids, liposomes, cationic polymers, micelles, emulsions, and solid lipid nanoparticles.

As yet another exemplary embodiment of the present invention, the IL-10 protein may be an amino acid sequence represented by SEQ ID NO: 1.

As yet another exemplary embodiment of the present invention, the IL-10 encoding gene may be a base sequence represented by SEQ ID NO: 2 or 3.

As yet another exemplary embodiment of the present invention, the GDNF protein may be an amino acid sequence represented by SEQ ID NO: 4.

As yet another exemplary embodiment of the present invention, the GDNF protein encoding gene may be a base sequence represented by SEQ ID NO: 5 or 6.

As yet another exemplary embodiment of the present invention, the GAD may be GAD 65 or GAD 67.

As yet another exemplary embodiment of the present invention, the GAD protein may be an amino acid sequence represented by SEQ ID NO: 7 or 9.

As yet another exemplary embodiment of the present invention, the GAD encoding gene may be a base sequence represented by SEQ ID NO: 8 or 10.

As yet another exemplary embodiment of the present invention, the degenerative brain disease may be at least one selected from the group consisting of Parkinson's disease, dementia, Alzheimer's disease, Huntington's disease, frontotemporal lobar degeneration, dementia with Lewy bodies, vascular dementia, corticobasal degeneration, multiple system atrophy, progressive supranuclear palsy, primary lateral sclerosis, spinal muscular atrophy, stroke, cerebral infarction, head trauma, spinal cord injury, cerebral arteriosclerosis, Lou Gehrig's disease, and multiple sclerosis disease.

As yet another exemplary embodiment of the present invention, the pharmaceutical composition may additionally contain a physiologically acceptable carrier.

As yet another exemplary embodiment of the present invention, the pharmaceutical composition may be an injection.

As yet another exemplary embodiment of the present invention, the pharmaceutical composition may have an effect of ameliorating motor abnormalities caused by neuronal cell death.

As yet another exemplary embodiment of the present invention, the neurons may be motor neurons, interneurons or sensory neurons.

As yet another exemplary embodiment of the present invention, the pharmaceutical composition may have an effect of improving cognitive ability and memory.

Further, the present invention provides a health functional food for preventing or ameliorating degenerative brain diseases, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

In addition, the present invention provides a method for preventing or treating degenerative brain diseases, the method including administering at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same to an individual in need thereof.

Furthermore, the present invention provides a use of at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same for preparing a preventive or therapeutic agent for degenerative brain diseases.

### [Advantageous Effects]

The composition of the present invention contains at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same. Therefore, since the pharmaceutical composition of the present invention exhibits a better effect of alleviating and preventing degenerative brain diseases by co-administration of active ingredients than upon single administration, the pharmaceutical composition of the present invention can exhibit the same or higher effect only with a low concentration of the active ingredients. Thus, the pharmaceutical composition of the present invention can be advantageously used for the prevention or treatment of degenerative brain diseases.

However, the effects of the present invention are not limited to the aforementioned effects and should be understood to include all possible effects deduced from the configuration of the invention described in the detailed description or the claims of the present invention.

### [Description of Drawings]

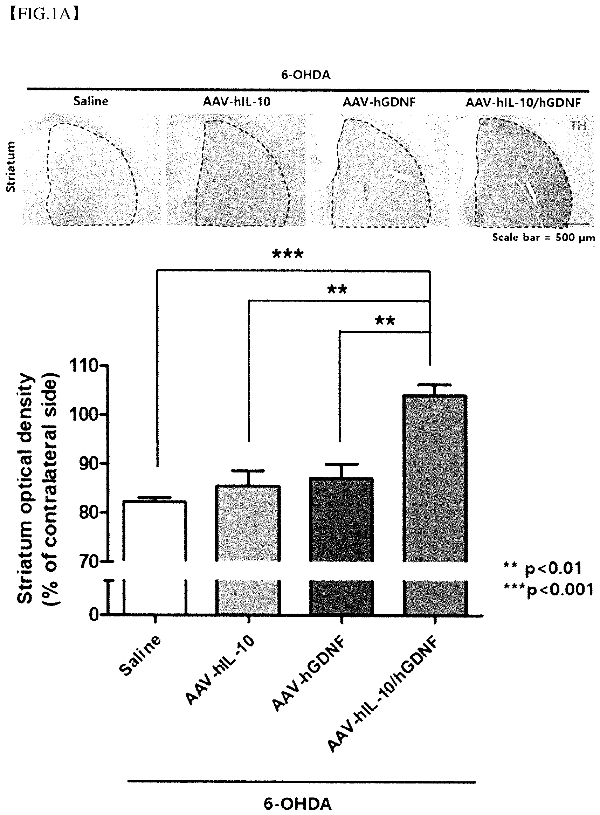

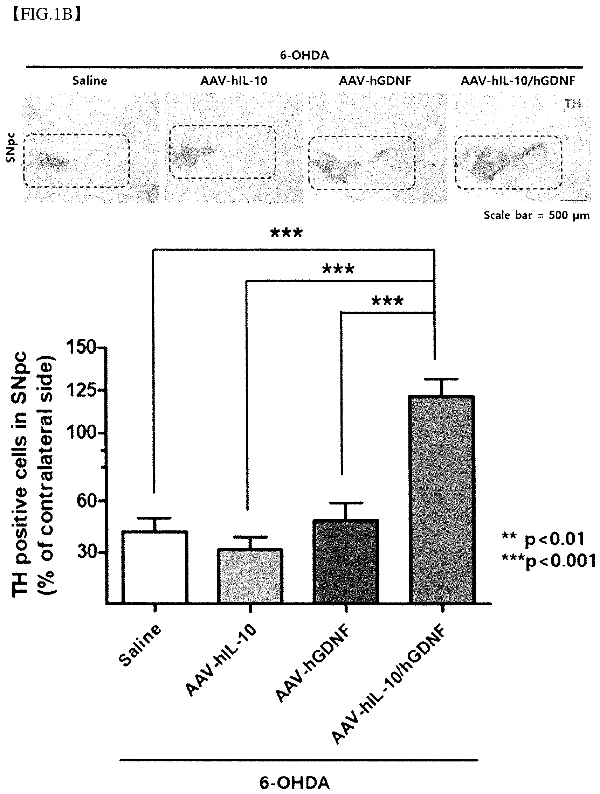

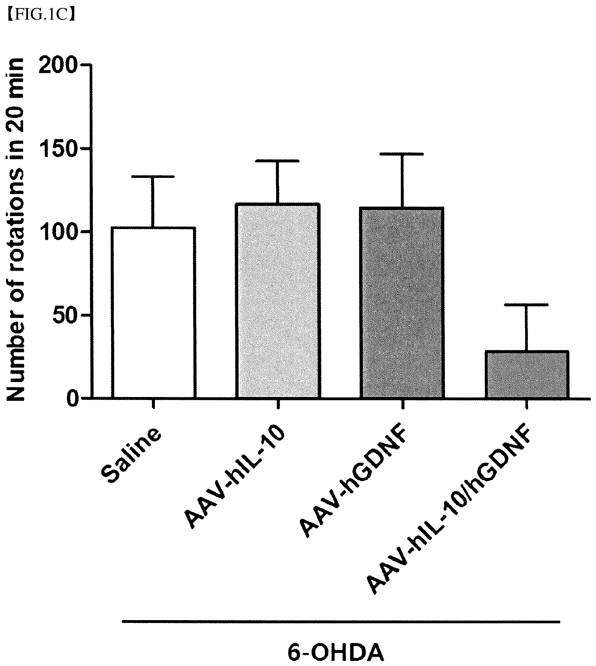

FIG. 1 is a set of views confirming the effect of co-administration of hIL-10 and hGDNF on ameliorating degenerative brain diseases, FIGS. 1A and 1B are views confirming that, in a degenerative brain disease animal model pretreated with test materials and induced by administration of 6-OHDA, rAAV5-hIL-10/hGDNF administration (co-administration) is more effective in protecting dopaminergic neurons than the administration of rAAV5-hIL-10 and rAAV5-hGDNF alone in the striatum region of the brain, FIG. 1B is a view confirming that, in a degenerative brain disease animal model pretreated with test materials and induced by administration of 6-OHDA, rAAVS-hIL-10/hGDNF administration (co-administration) is more effective in protecting dopaminergic neurons than the administration of rAAV5-hIL-10 and rAAV5-hGDNF alone in the substantia nigra pars compacta (SNpc) region, and FIG. 1C is a view confirming, through a rotation test, that rAAV5-hIL-10/hGDNF administration (co-administration) is more effective in ameliorating hypokinesia symptoms than the administration of rAAV5-hIL-10 and rAAV5-hGDNF alone.

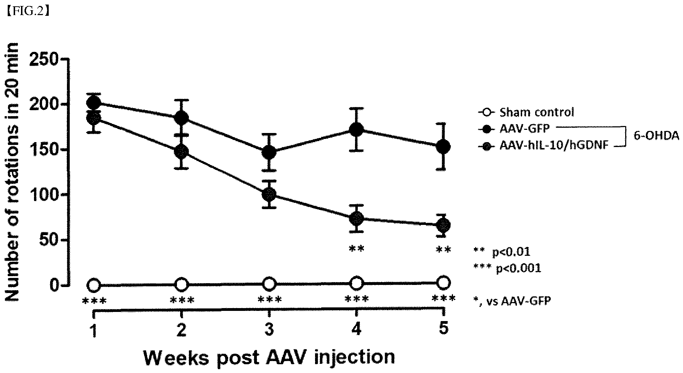

FIG. 2 is a view confirming the effect of ameliorating long-term motor function in a degenerative brain disease through a rotation test after administering 6-OHDA to produce an animal model of Parkinson's disease, and then administering rAAV5-hIL-10/hGDNF in order to confirm the effect of rAAV5-hIL-10/hGDNF on the amelioration of persistent degenerative brain disease.

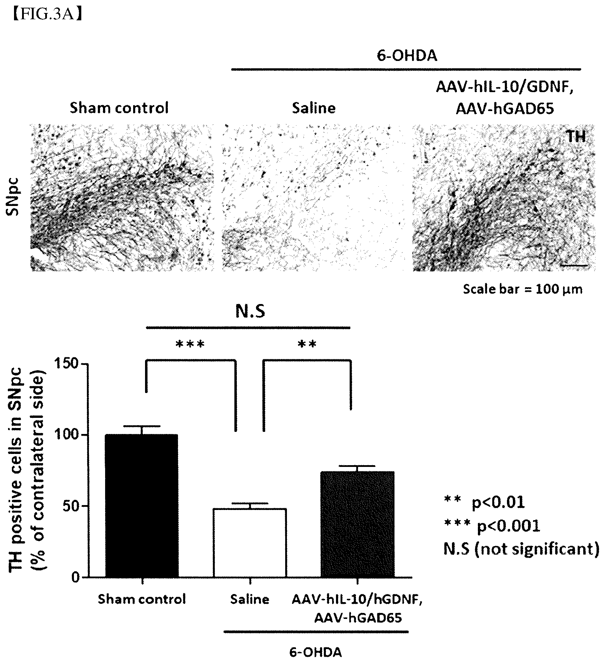

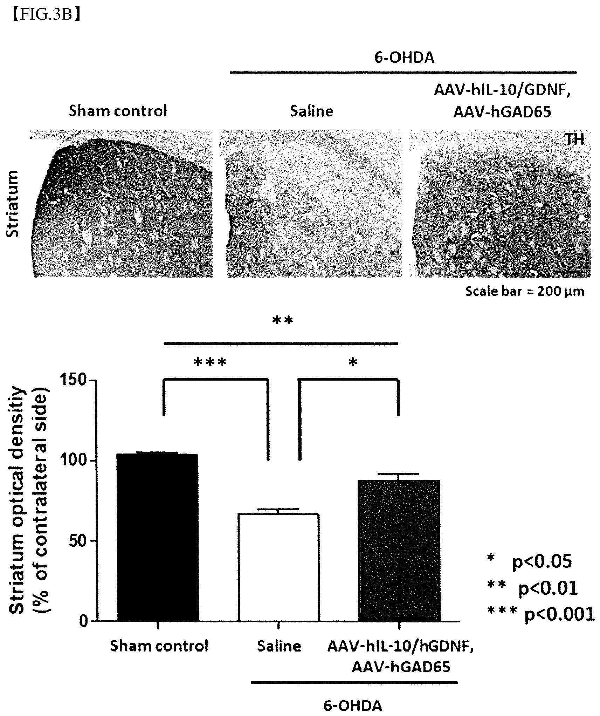

FIG. 3 is a set of views confirming the neuroprotective effect of the gene therapeutic agent of the present invention on dopaminergic neurons by pretreating mice with one of the gene therapeutic agents of the present invention (rAAV5-hIL-10/hGDNF and rAAV5-hGAD65) and then treating the mice with 6-OHDA to produce a degenerative brain disease animal model. Specifically, FIG. 3A shows the result confirmed in the striatum, and FIG. 3B shows the result confirmed in the substantia nigra pars compacta (SNpc).

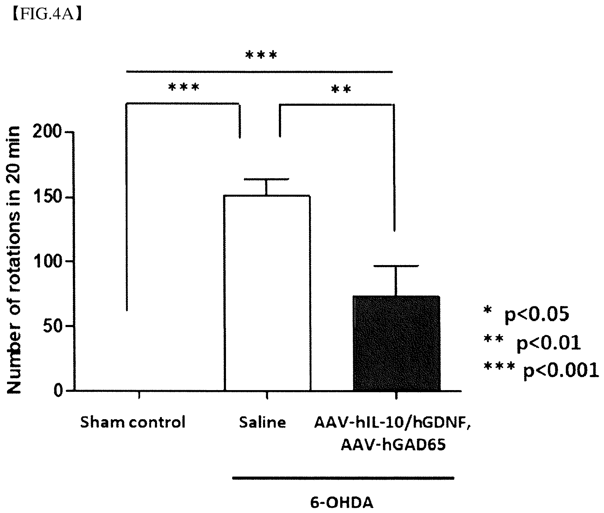

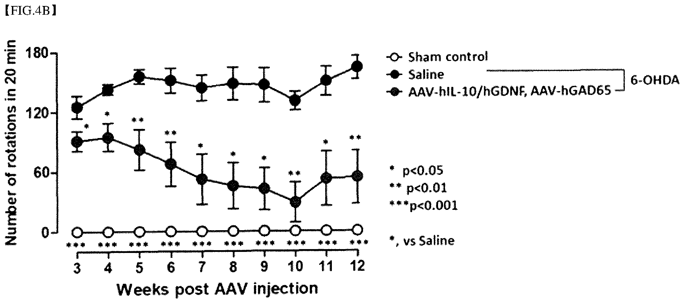

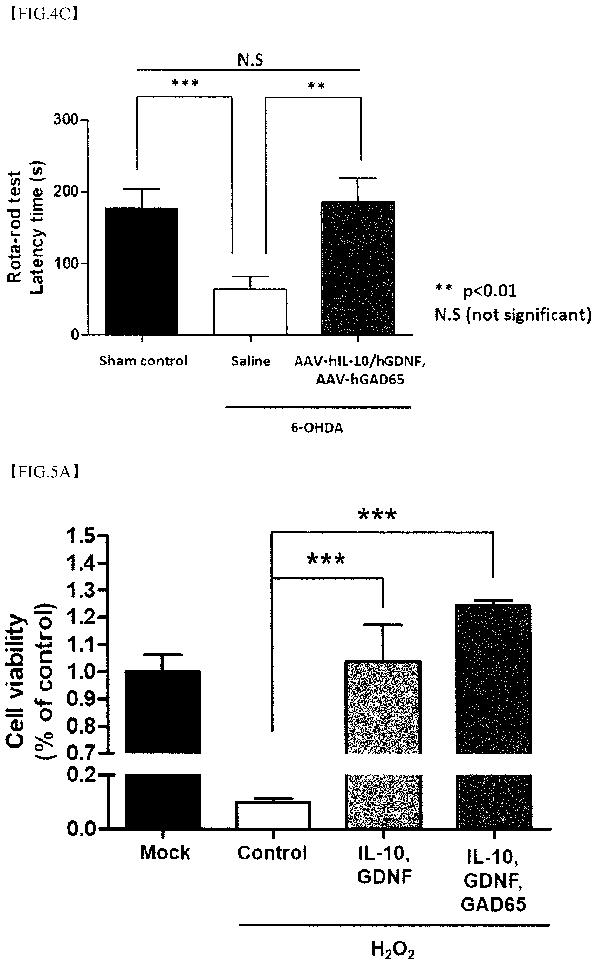

FIG. 4 confirms the effect of the gene therapeutic agent of the present invention on the prevention of hypokinesia by pretreating mice with one of the gene therapeutic agents of the present invention (rAAV5-hIL-10/hGDNF and rAAV5-hGAD65), and then treating the mice with 6-OHDA to produce a degenerative brain disease animal model, and specifically, FIG. 4A confirms the results of a rotation test 4 weeks after producing the mouse model, FIG. 4B shows the results of the rotation test observed continuously for 10 weeks after producing the mouse model, and FIG. 4C shows the results of confirming the results of Rota-rod 2 weeks after the production of the mouse model.

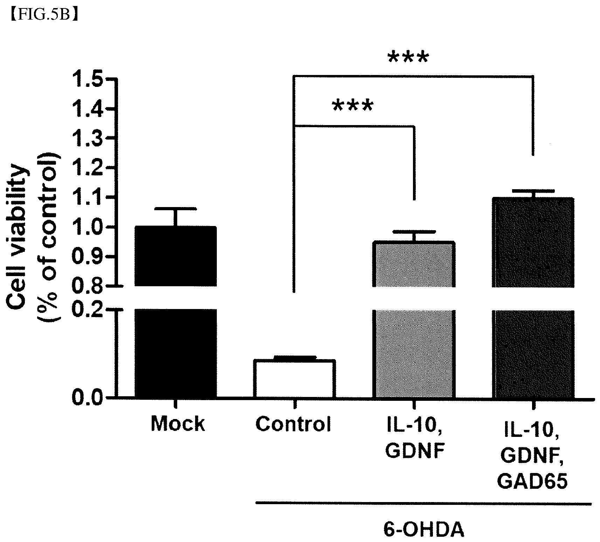

FIG. 5 is a set of views confirming the effect of inhibiting neuronal cell death caused by oxidative stress upon co-administration of protein cultures of hIL-10 and hGDNF produced by plasmids or simultaneous administration of protein cultures of hIL-10, hGDNF and hGAD65, and specifically, FIG. 5A and FIG. 5B are views confirming that the effect of inhibiting neuronal cell death is exhibited upon co-administration of protein cultures of hIL-10 and hGDNF produced by plasmids or simultaneous administration of protein cultures of hIL-10, hGDNF and hGAD65 in the case of neuronal cell death by H₂O₂ and in the case of neuronal cell death by 6-OHDA, respectively.

### [Modes of the Invention]

The present inventors conducted intensive studies to develop a therapeutic agent having preventive or therapeutic effects against degenerative brain diseases, and as a result, confirmed that when at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same are used, the case shows an excellent preventive or therapeutic effect on degenerative brain diseases compared to a use of only one, thereby completing the present invention.

Thus, the present invention provides a pharmaceutical composition for preventing or treating degenerative brain diseases, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

As used herein, the term "prevention" refers to all actions that suppress or delay the onset of a degenerative brain disease by administering the pharmaceutical composition provided by the present invention to an individual expected to develop the degenerative brain disease.

As used herein, the term "treatment" refers to all actions that clinically intervene to alter the natural processes of the individual or cells to be treated, and may be carried out either during the development of a clinical pathological condition or to prevent the clinical pathological condition. The desired therapeutic effect includes preventing the occurrence or recurrence of a disease, alleviating symptoms, reducing all direct or indirect pathological consequences of the disease, preventing metastasis, reducing a disease progression rate, mitigating or temporarily alleviating a condition, making a patient feel better, or improving the prognosis. For the purpose of the present invention, the treatment may be interpreted as including all actions of administering the pharmaceutical composition of the present invention to a patient who has developed a degenerative brain disease to ameliorate the symptoms of the degenerative brain disease, but is not limited thereto.

A combination of the two or more may be interleukin 10 (IL-10) and glial cell line-derived neurotrophic factor (GDNF); interleukin 10 (IL-10) and glutamate decarboxylase (GAD); glial cell line-derived neurotrophic factor (GDNF) and glutamate decarboxylase (GAD); interleukin 10 (IL-10), glial cell line-derived neurotrophic factor (GDNF) and glutamate decarboxylase (GAD), and the IL-10, GDNF and GAD may be each a protein or gene encoding the same.

Further, the gene may be DNA or RNA (specifically mRNA), and may be in the form of being operably contained in a vector. Specifically, the gene may be in the form of being operably included in a viral vector or a non-viral vector.

The gene encoding IL-10 may be operably included in a first vector, the gene encoding GDNF may be operably included in a second vector, the gene encoding GAD may be operably included in a third vector, and two or more genes may be included in one vector.

The two or more genes selected from the group consisting of the gene encoding IL-10, the gene encoding GDNF, and the gene encoding GAD may be in the form of being included in a vehicle. In this case, the vehicle may be a viral vector, or a non-viral vector such as a plasmid or a liposome. In addition, some of the genes may be in the form of being included in a viral vector and the remaining genes may be included in the form of being included in a non-viral vector.

As an exemplary embodiment, IL-10 may be in the form of being included in a viral vector, and GDNF may be in the form of being included in a non-viral vector. Furthermore, GDNF may be in the form of being included in a viral vector, and IL-10 may be in the form of being included in a non-viral vector. Moreover, IL-10 may be in the form of being included in a viral vector, and GAD may be in the form of being included in a non-viral vector. Further, IL-10 may be in the form of being included in a viral vector, and GDNF and GAD may be in the form of being included in a non-viral vector. In addition, IL-10 and GDNF may be in the form of being included in a viral vector, and GAD may be in the form of being included in a non-viral vector. Furthermore, IL-10 and GAD may be in the form of being included in a viral vector, and GDNF may be in the form of being included in a non-viral vector. Further, IL-10 may be in the form of being included in a viral vector, and GDNF and GAD may be in the form of being included in a non-viral vector. In addition, GDNF and GAD may be in the form of being included in a viral vector, and IL-10 may be in the form of being included in a non-viral vector. Furthermore, GDNF may be in the form of being included in a viral vector, and IL-10 and GAD may be in the form of being included in a non-viral vector. Further, all of IL-10, GDNF and GAD may be in the form of being included in a viral vector. All of IL-10, GDNF and GAD may be in the form of being included in a non-viral vector.

The viral vector may be at least one selected from the group consisting of adenoviruses, adeno-associated viruses (AAV), herpes simplex virus, lentiviruses, retroviruses, cytomegalovirus, baculoviruses, poxviruses, and the like, but is not limited thereto. Preferably, the viral vector may be an adeno-associated virus (AAV).

In addition, the non-viral vector may be at least one selected from the group consisting of plasmids, liposomes, cationic polymers, micelles, emulsions, and solid lipid nanoparticles.

As used herein, the term "plasmid" refers to a circular DNA fragment that is present separately from the bacterial chromosome. The plasmid does not include genes that are essential for bacterial survival, but it includes genes that are essential for resistance to certain antibiotics and gene exchange between bacteria. Furthermore, the plasmid can proliferate independently of chromosomes, and may include a selectable marker.

As used herein, the term "liposome" refers to a vesicle formed while forming a bilayer due to a hydrophilic part and a hydrophobic part when molecules having both the hydrophobic part and the hydrophilic part, such as phospholipids, are suspended in an aqueous solution. Liposomes are separated from the outer membrane by a membrane composed of a lipid bilayer, and liposomes incorporating DNA, mRNA, and the like may be used as carriers of genetic information.

As used herein, the term "cationic polymer" refers to a material that forms a complex with anionic DNA through ionic bonding as a cationic lipid or polymer compound, and is delivered into cells.

As used herein, the term "micelle" refers to a thermodynamically stable colloidal aggregate made by the association of molecules consisting of polar groups and non-polar groups, such as surfactants and lipid molecules, by van der Waals forces in a solution. Further, micelles containing DNA, mRNA, and the like may be used as carriers of genetic information.

As used herein, the term "emulsion" means that, when two solutions having different phases are mixed, one liquid forms fine particles and is dispersed in the other liquid. DNA, mRNA and the like may be included in the core of the emulsion particles and used as carriers of genetic information.

As used herein, the term "solid lipid nanoparticles" refers to a formulation in which a drug is incorporated in nano-sized microparticles consisting of a solid lipid instead of a liquid lipid.

A mixing ratio of Vehicle 1 (for example, a first vector) including any one gene selected from the group consisting of IL-10, GDNF and GAD and Vehicle 2 (for example, a second vector) including at least one gene selected from the group consisting of the remaining genes, which are not included in Vehicle 1, may be 1:1 to 100 or 1 to 100:1. Specifically, although not limited thereto, the mixing ratio of Vehicle 1 and Vehicle 2 may be 1:1 to 100 or 1 to 100:1 based on VG.

Vehicle 1 (for example, a first vector) including the gene encoding IL-10, Vehicle 2 (for example, a second vector) including the gene encoding GDNF, and Vehicle 3 (for example, a third vector) including the gene encoding GAD may be mixed in various ratios, and for example, the mixing ratio may be 1:1:1 to 1:1:100; 1:1:1 to 1:100:1; 1:1:1 to 100:1:1; 1:1:1 to 1:100:100; 1:1:1 to 100:100:1; or 1:1:1 to 100:1:100, but is not limited thereto.

As used herein, the term "operably" refers to being linked to a regulatory sequence in a manner that allows a transgene to be expressed in a host cell. The regulatory sequence is a DNA sequence that regulates gene expression, and may include promoters and other regulatory elements such as enhancers and polyadenylation. In addition, the regulatory sequence provides a binding site for the transcription factor that regulates the expression of the transgene, and may affect a complex structure with the transcription factor to determine the function of the transcription factor.

As used herein, the term "IL-10" refers to an anti-inflammatory cytokine belonging to class II cytokines (Renauld, Nat Rev Immunol, 2003). IL-10 is in the form of a homodimer consisting of two subunits, each with a length of 178 amino acids, and is also known as a human cytokine synthesis inhibitory factor (CSIF). IL-10 performs the function of suppressing the activity of natural killer (NK) cells in immune responses and forms a complex with the IL-10 receptor to participate in signal transduction. IL-10 may be a human-, mouse-, rat-, dog-, cat-, or horse-derived protein or a base sequence encoding the same, but is not limited thereto. IL-10 may consist of an amino acid sequence of SEQ ID NO: 1 of NCBI Accession No.NP_000563.1, and the gene encoding the same may be a base sequence represented by SEQ ID NO: 2 or SEQ ID NO: 3.

The IL-10 encoding gene may be a base sequence encoding an IL-10 variant that maintains IL-10 activity. The base sequence encoding the IL-10 variant may be a base sequence encoding an amino acid sequence having a sequence homology of at least 60% or more, 70% or more, 80% or more, or 90% or more with the IL-10 amino acid sequence indicated above, and may be most preferably a base sequence encoding an amino acid sequence having a sequence homology of 95% or more.

Furthermore, the base sequence encoding the IL-10 variant may be a base sequence having a sequence homology of at least 60% or more, 70% or more, 80% or more, or 90% or more with the IL-10 base sequence indicated above, and may be most preferably a base sequence having a sequence homology of 95% or more.

The "% sequence homology" is confirmed by comparing comparison regions with two sequences optimally aligned, and a portion of the base sequence in the comparison region may include an addition or deletion (that is, a gap) compared to the reference sequence (without addition or deletion) for the optimal alignment of the two sequences.

As used herein, the term "GDNF" refers to one nerve growth factor that constitutes the GDNF ligand family, and the GDNF ligand family includes GDNF, neuturin (NRTN), artemin (ARTN), and persephin (PSPN). Further, GDNF is a protein that promotes the survival of many types of neurons, and transmits signals through GDNF receptor alpha-1 (GFR α1), GDNF receptor alpha-2 (GFR α2) and GDNF receptor alpha-3 (GFR α3). GDNF may be a human-, mouse-, rat-, dog-, cat-, or horse-derived protein or a base sequence encoding the same, but is not limited thereto. GDNF may consist of an amino acid sequence of SEQ ID NO: 4 of NCBI Accession No. NCBI NP_954701.1, and the gene encoding the same may be a base sequence represented by SEQ ID NO: 5 or SEQ ID NO: 6.

The GDNF encoding gene may be a base sequence encoding a GDNF variant that maintains GDNF activity. The base sequence encoding the GDNF variant may be a base sequence encoding an amino acid sequence having a sequence homology of at least 60% or more, 70% or more, 80% or more, or 90% or more with the GDNF amino acid sequence indicated above, and may be most preferably a base sequence encoding an amino acid sequence having a sequence homology of 95% or more.

Further, the base sequence encoding the GDNF variant may be a base sequence having a sequence homology of at least 60% or more, 70% or more, 80% or more, or 90% or more with the GDNF base sequence indicated above, and may be most preferably a base sequence having a sequence homology of 95% or more.

As used herein, the term "GAD" refers to an enzyme that decarboxylates glutamic acid to produce gamma-aminobutyric acid (GABA). The GAD may be GAD65 or GAD67. Specifically, the GAD65 may be a human-, mouse-, rat-, dog-, cat-, or horse-derived protein or a base sequence encoding the same, but is not limited thereto. The GAD65 may consist of an amino acid sequence of SEQ ID NO: 7 of NCBI Accession No. NP_000809.1, and the gene encoding the same may be a base sequence represented by SEQ ID NO: 8. The GAD67 may be a human-, mouse-, rat-, dog-, cat-, or horse-derived protein or a base sequence encoding the same, but is not limited thereto. The GAD67 may consist of an amino acid sequence of SEQ ID NO: 9 of NCBI Accession No. NP_000808.2, and the gene encoding the same may be a base sequence represented by SEQ ID NO: 10.

In addition, the GAD encoding gene may be a base sequence that encodes a GAD variant that enables GABA to be produced by maintaining GAD activity. The GAD variant includes all sequences in which the characteristics of GAD producing GABA are maintained, and is not limited to any one, but preferably, the base sequence encoding the GAD variant may be a base sequence encoding an amino acid sequence having a sequence homology of at least 60% or more, 70% or more, 80% or more, or 90% or more with the GAD amino acid sequence indicated above, and may be most preferably a base sequence encoding an amino acid sequence having a sequence homology of 95% or more.

In addition, the base sequence encoding the GAD variant may be a base sequence having a sequence homology of at least 60% or more, 70% or more, 80% or more, or 90% or more with the GAD base sequence indicated above, and may be most preferably a base sequence having a sequence homology of 95% or more.

The pharmaceutical composition of the present invention may exhibit a preventive effect against degenerative brain diseases through a gene or a vehicle including the gene. The composition of the present invention may achieve a better degenerative brain disease preventive or therapeutic effect than single administration by co-administering materials having different mechanisms.

In particular, according to the present invention, when two or more genes selected from the group consisting of genes encoding IL-10, GDNF and GAD65 are co-administered, a synergistic preventive or therapeutic effect on degenerative brain diseases occurs. Therefore, the pharmaceutical composition of the present invention may be advantageously used for the prevention or treatment of degenerative brain diseases, and specifically may ameliorate motor abnormalities in degenerative brain diseases caused by the death of neurons, and may ameliorate cognitive and memory decline, which are the main symptoms of degenerative brain diseases.

In the present invention, each of the vectors including IL-10, GDNF and GAD65 may be administered alone or two or more thereof may be administered together, and may also be administered in a form including two or more genes in one vector. As an administration site, IL-10 may be administered to the striatum (ST), GDNF may be administered to the striatum (ST), and GAD65 may be administered to the subthalamic nucleus (STN), but the administration is not limited thereto. The administration site of rAAV5-hGDNF/hIL-10, which is one of the aforementioned examples, may be the striatum (ST), but is not limited thereto.

In the present invention, the neurons may be motor neurons that transmit stimuli to the brain or spinal cord to transmit the stimuli to effectors such as muscle cells or secretory glands, interneurons that are distributed throughout the central nervous system and transmit stimuli from sensory neurons to motor neurons, or sensory neurons that transmit stimuli to the brain or spinal cord to transmit the stimuli to effectors such as muscle cells or secretory glands.

In the present invention, the degenerative brain disease is not limited to, but may be at least one selected from the group consisting of Parkinson's disease, dementia, Alzheimer's disease, Huntington's disease, frontotemporal lobar degeneration, dementia with Lewy bodies, vascular dementia, corticobasal degeneration, multiple system atrophy, progressive supranuclear palsy, primary lateral sclerosis, spinal muscular atrophy, stroke, cerebral infarction, head trauma, spinal cord injury, cerebral arteriosclerosis, Lou Gehrig's disease, and multiple sclerosis disease, and may be preferably Parkinson's disease, dementia or Alzheimer's disease.

The present inventors confirmed, through specific exemplary embodiments, that the pharmaceutical composition of the present invention can be advantageously used for the prevention or treatment of degenerative brain diseases.

In an exemplary embodiment of the present invention, it was confirmed that, when rAAV5-hIL-10/hGDNF was administered to a mouse model in which a degenerative brain disease was induced, motor dysfunction, which is a symptom induced by the degenerative brain disease, was ameliorated to an effective level (see Example 1).

In another exemplary embodiment of the present invention, it was confirmed that when 6-OHDA was injected into a mouse model to induce a degenerative brain disease after pretreatment with rAAV5-hIL-10/hGDNF and rAAV5-hGAD65, dopaminergic neuronal cell death and motor dysfunction, which are symptoms induced by degenerative brain diseases, were ameliorated when the pharmaceutical composition of the present invention was administered (see Example 2).

In still another embodiment of the present invention, it was confirmed that in the case of pretreatment with hIL-10 and hGDNF protein cultures produced by plasmids or protein cultures of hIL-10, hGDNF and hGAD65, neuronal cell death induced by H₂O₂ or 6-OHDA was suppressed (see Example 3).

Through the aforementioned results, the present inventors were able to confirm that degenerative brain diseases can be prevented or treated when the pharmaceutical composition of the present invention is administered.

The pharmaceutical composition of the present invention may further include a suitable carrier, excipient, and diluent, which are typically used to prepare a pharmaceutical composition. A composition including a pharmaceutically acceptable carrier may be in the form of various oral or parenteral formulations. When the composition is formulated, the composition may be prepared using a commonly used diluent or excipient, such as a filler, an extender, a binder, a wetting agent, a disintegrant, and a surfactant. A solid formulation for oral administration may include a tablet, a pill, a powder, a granule, a capsule, and the like, and the solid formulation may be prepared by mixing at least one excipient, for example, starch, calcium carbonate, sucrose or lactose, gelatin, and the like with one or more compounds.

Furthermore, in addition to simple excipients, lubricants such as magnesium stearate and talc may also be used. A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, an aromatic, a preservative, and the like. A formulation for parenteral administration may include an aqueous sterile solution, a non-aqueous solvent, a suspension, an emulsion, a freeze-dried preparation, and a suppository. As the non-aqueous solvent and the suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerogelatin, and the like.

Further, the pharmaceutical composition of the present invention may have any one formulation selected from the group consisting of a tablet, a pill, a powder, a granule, a capsule, a suspension, a liquid for internal use, an emulsion, a syrup, a sterilized aqueous solution, a non-aqueous solvent, a suspension, an emulsion, a freeze-dried preparation, and a suppository.

Meanwhile, as another aspect of the present invention, the present invention provides a method for preventing or treating degenerative brain diseases, the method including administering the pharmaceutical composition to an individual.

As used herein, the term "individual" refers to all animals, including humans, who are likely to develop or have developed the degenerative brain disease. By administering the compositions of the present invention to an individual, symptoms induced by a degenerative brain disease may be alleviated or treated.

As used herein, the term "alleviation" refers to all actions that ameliorate or beneficially change symptoms of a degenerative brain disease by administering the pharmaceutical composition according to the present invention.

The composition of the present invention is administered in a pharmaceutically effective amount.

As used herein, the term "administration" refers to introduction of the pharmaceutical composition of the present invention into a subject by any appropriate method, and for the administration route, the pharmaceutical composition of the present invention may be administered via various routes, either oral or parenteral, as long as the pharmaceutical composition of the present invention may reach a target tissue.

The pharmaceutical composition of the present invention may be appropriately administered to an individual according to the typical method, administration route and dosage used in the art, according to the purpose or need. Examples of the administration route include oral, parenteral, subcutaneous, intraperitoneal, intrapulmonary, and intranasal administration, and parenteral injection includes intramuscular, intravenous, intraarterial, intraperitoneal, or subcutaneous administration.

In addition, an appropriate dosage and administration frequency may be selected according to methods known in the art, and the amount and administration frequency of the pharmaceutical composition of the present invention actually administered may be appropriately determined by various factors such as the type of symptoms to be treated, administration route, sex, health status, diet, age and weight of the individual and severity of disease.

In the case of a viral vector, the administration frequency may be once or more per year, and when the viral vector is repeatedly administered, it may be administered at intervals of 1 day to 1 month, or 1 month to 1 year. Furthermore, when the pharmaceutical composition is a non-viral vector, it may be administered once or more a year, and when the non-viral vector is repeatedly administered, it may be administered at intervals of 12 to 24 hours and 1 to 14 days. Further, when the pharmaceutical composition is a protein, it may be administered once or more a year, and when it is repeatedly administered, it may be administered at intervals of 1 day to 1 month, or 1 month to 1 year.

As used herein, the term "pharmaceutically effective amount" refers to an amount sufficient to treat a degenerative brain disease at a reasonable benefit/risk ratio applicable to medical use, and an effective dosage level may be determined according to factors including the type of individual, the severity of disease, age, sex, the activity of drugs, sensitivity to drugs, administration time, administration route, excretion rate, treatment period, and simultaneously used drugs, and other factors well known in the medical field.

The pharmaceutical composition of the present invention may be administered as an individual therapeutic agent or may be administered in combination with other therapeutic agents, and may be administered sequentially or simultaneously with therapeutic agents in the related art. Also, the composition of the present invention may be administered in a single dose or multiple doses. It is important to administer the composition in a minimum amount that can obtain the maximum effect without any side effects, in consideration of all the aforementioned factors, and this amount may be easily determined by those skilled in the art.

Further, the present invention provides a health functional food for preventing or ameliorating degenerative brain diseases, containing at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

The "interleukin 10," "glial cell line-derived neurotrophic factor," "glutamate decarboxylase," "protein," "gene," "degenerative brain disease," "prevention," and the like may fall within the above-described ranges.

The term "amelioration" may mean all actions that at least reduce a parameter associated with the condition to be treated, for example, the severity of symptoms. In this case, in order to prevent or alleviate a degenerative brain disease, the health functional food may be used simultaneously with or separately from a drug for treatment before or after the onset of the corresponding disease.

The health functional food may be used by adding an active ingredient as it is to food or may be used together with other foods or food ingredients, and may be appropriately used according to a typical method. The mixing amount of the active ingredient may be suitably determined depending on its purpose of use (for prevention or alleviation). In general, the health functional food may be added in an amount of about 15% by weight or less, more specifically about 10% by weight or less, based on the raw materials when producing food or beverages. However, for long-term intake for the purpose of health and hygiene or for the purpose of health control, the amount may be below the above-mentioned range.

The health functional food further includes one or more of a carrier, a diluent, an excipient, and an additive, and thus, may be formulated into one selected from the group consisting of a tablet, a pill, a powder, a granule, a powder, a capsule, and a liquid formulation. Foods to which the compound according to an aspect can be added include various foods, powders, granules, tablets, capsules, syrups, beverages, gums, teas, vitamin complexes, health functional foods, and the like.

Specific examples of the carrier, excipient, diluent, and additive may be one or more selected from the group consisting of lactose, dextrose, sucrose, sorbitol, mannitol, erythritol, starch, acacia rubber, calcium phosphate, alginate, gelatin, calcium phosphate, calcium silicate, microcrystalline cellulose, polyvinylpyrrolidone, cellulose, polyvinylpyrrolidone, methyl cellulose, water, sugar syrup, methyl cellulose, methyl hydroxybenzoate, propyl hydroxybenzoate, talc, magnesium stearate, and mineral oil.

In addition to containing the active ingredient, the health functional food may contain other ingredients as essential ingredients without any particular limitation. For example, the health functional food may contain various flavoring agents or natural carbohydrates, and the like as an additional ingredient, as in a typical beverage. Examples of the above-described natural carbohydrates include typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavoring agent other than those mentioned above, a natural flavoring agent (thaumatin, a stevia extract (for example, rebaudioside A, glycyrrhizin and the like)), and a synthetic flavoring agent (saccharin, aspartame and the like) may be advantageously used. The proportion of the natural carbohydrate may be appropriately determined by the choice of a person skilled in the art.

The health functional food according to an aspect may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in carbonated beverages, or the like, in addition to the additives mentioned above. Such components may be used independently or in combination, and the proportion of these additives may also be appropriately selected by those skilled in the art.

In addition, the present invention provides a method for preventing or treating degenerative brain diseases, the method including administering at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same to an individual in need thereof.

The "interleukin 10," "glial cell line-derived neurotrophic factor," "glutamate decarboxylase," "protein," "gene," "degenerative brain disease," "prevention," "treatment," "individual" and the like may fall within the above-described ranges.

Furthermore, the present invention provides a use of at least two selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same for preparing a preventive or therapeutic agent for degenerative brain diseases.

The "interleukin 10," "glial cell line-derived neurotrophic factor," "glutamate decarboxylase," "protein," "gene," "degenerative brain disease," "prevention," "treatment" and the like may fall within the above-described ranges.

Hereinafter, preferred examples for helping with understanding of the present invention will be suggested. However, the following examples are provided only so that the present invention may be more easily understood, and the content of the present invention is not limited by the following examples.

### [Examples]

### Example 1. Confirmation of synergistic effect of co-administration of hIL-10 and hGDNF on amelioration of degenerative brain disease

To confirm the effect on a degenerative brain disease when rAAV5-hIL-10/hGDNF, which is one of the gene therapeutic agents of the present invention, is administered, saline, rAAV5-hIL-10, rAAV5-hGDNF and rAAV5-hIL-10/hGDNF were administered to animal models of Parkinson's disease to confirm their ameliorative effect on a degenerative brain disease through the suppression of dopaminergic neuronal cell death and the improvement of motor function.

### 1-1. Preparation of animal model

It is known that the brains of animals or humans with a degenerative brain disease show a decline in motor ability due to changes in signal transmitters of dopaminergic neurons and the like compared to the brains of normal animals or humans. An animal model for confirming the effect of the gene therapeutic agent of the present invention on ameliorating degenerative brain diseases was produced using a mouse (C57BL/6 mouse). To produce a degenerative brain disease mouse model, 6-hydroxydopamine (6-OHDA), which is known to induce dopaminergic neuronal cell death and abnormal excitatory neurotransmission in the subthalamic nucleus (STN), was administered into the left striatum (ST) of the mouse. A specific method for producing a degenerative brain disease mouse model is as follows.

The mice were anesthetized by intraperitoneal administration of an Alfaxan-sedator complex, the head of the anesthetized animal was fixed using the nose clip and ear bars of a stereotaxic instrument (RWD Life Science), and then the height of the bregma and lambda of the mouse was measured to adjust the position such that the head was horizontal. After the coordinates of a desired brain position were confirmed based on the bregma, a hole was made in the brain bone at the confirmed position using a micromotor drill (Seashin). A material (may be saline, 0.1% ascorbic acid, 6-hydroxydopamine (OHDA), or rAAV5-GFP, and may vary depending on each experiment) to be administered was put into a Hamilton syringe (Hamilton) (a 10-µL syringe was used, but is not limited thereto), and about 2 µL of the material was injected into the brain coordinate position at a rate of 0.5 µL/min using a stereotaxic injection pump (Dongbang Hitech Inc.). Mice to which the substance was administered had their scalp sutured using black silk, were disinfected with povidone, returned to their cages, and exposed to infrared heat such that their body temperature could be maintained until the anesthesia completely wore off.

### 1-2. Confirmation of protective effect of co-administration of hIL-10 and hGDNF on dopaminergic neurons in animal model

Specifically, in order to confirm the effect of saline, rAAV5-hIL-10, rAAV5-hGDNF and rAAV5-hIL-10/hGDNF on the suppression of dopaminergic neuronal cell death, after pretreating the mice with saline, rAAV5-hIL-10, rAAV5-hGDNF and rAAV5-hIL-10/hGDNF, 2 weeks later, a Parkinson's disease animal model was produced by administering 6-OHDA. 3,3'-diaminobenzidine staining (DAB staining) was performed to confirm the protective effect on dopaminergic neurons in the striatum (ST) in which the axons of the substantia nigra pars compacta (SNpc) and SNpc cells extend. In the experiment, 6 weeks after administration of saline, rAAV5-hIL-10, rAAV5-hGDNF and rAAV5-hIL-10/hGDNF (4 weeks after administration of 6-OHDA), the mice were sacrificed to obtain brain tissue, sectioned tissue was treated with 1% H₂O₂ for 15 minutes in order to prevent nonspecific color development, and then to detect tyrosine hydroxylase (TH), which is known as a representative dopaminergic neuronal marker, the sectioned tissue was treated with an anti-TH antibody and left at a temperature of 4°C for 8 hours or more. The tissue was treated with a biotinylated antibody to adhere to the anti-TH antibody, an avidin-biotin enzyme conjugate was bound to the biotinylated antibody using an ABC kit, and then the bound biotin was colored brown using a DAB kit and then observed under a microscope, under white light.

As a result, as shown in Fig. 1A, it could be confirmed that a group pretreated with rAAV5-hIL-10/hGDNF showed a significantly lower level of dopaminergic neuronal cell death in the striatum compared to groups pretreated with saline, rAAV5-hIL-10 or rAAV5-hGDNF alone, and further, even the case where a group was pretreated with rAAV5-hIL-10/hGDNF showed a significantly lower level of dopaminergic neuronal cell death in the SNpc compared to the case where a group was not pretreated with rAAV5-hIL-10/hGDNF (pretreated with saline, rAAV5-hIL-10, or rAAV5-hGDNF alone) (FIG. 1B).

### 1-3. Confirmation of amelioration effect of rAAV5-hIL-10/hGDNF on hypokinesia symptoms in degenerative brain disease animal model

In addition, a rotation test was performed to confirm the motor function improvement effects of saline, rAAV5-hIL-10, rAAV5-hGDNF and rAAV5-hIL-10/hGDNF. For the rotation test, 0.4 mg/mL apomorphine was dissolved in physiological saline, 4 mg/kg of apomorphine was subcutaneously administered to each degenerative brain disease mouse model, and after 5 minutes, the direction and degree of movement of the animal were recorded and captured using a camera for 20 minutes, and the recorded video was used to analyze the amount of clockwise rotation of the animal.

As a result, it could be confirmed that, when mice administered the gene therapeutic agent of the present invention (rAAV5-hIL-10/hGDNF) were compared with mice administered saline, rAAV5-hIL-10 or rAAV5-hGDNF, an effect of ameliorating a decline in motor function was shown (FIG. 1C).

### 1-4. Confirmation of therapeutic effect of rAAV5-hIL-10/hGDNF on persistent degenerative brain disease

A mouse model was produced by administering 6-OHDA to confirm whether the therapeutic effect of a degenerative brain disease persists when rAAV5-hIL-10/hGDNF, which is one of the gene therapeutic agents of the present invention, is administered, and after one week had passed, an effect of ameliorating a degenerative brain disease was confirmed by administering rAAV5-hIL-10/hGDNF. Specific experimental methods are shown in the following Table 1.

**[Table 1]**

| **Group** | **Animals** | **Injection materials** | |
|---|---|---|---|
| | | **6-OHDA lesion surgery** | **Test article injection** |
| **0.1 %AA+Saline** | ICR, male (8 weeks) | 2 µL 0.1% AA | ST: 2 µL Saline |
| **6-OHDA+rAAV5-GFP** | | 16 µg 6-OHDA/2 µL 0.1% AA | ST: rAAV5-GFP 1×10¹⁰ VG/2 µL |
| **6-OHDA+rAAV5-hIL-10/hGDNF** | | | ST: rAAV5-hIL-10/hGDNF 1×10¹⁰ VG/2 µL |

As a result of confirming the effect of ameliorating a decline in behavioral function of a degenerative brain disease when rAAV5-hGDNF/hIL-10 was performed was post-treated by performing a rotation test in the same manner as in Example 1-3 above, as shown in FIG. 2, it could be confirmed that even when rAAV5-hIL-10/hGDNF alone was administered, the decline in behavioral function induced by 6-OHDA was ameliorated, and that such an effect was maintained for a long period of time even after 5 weeks had passed after the administration of rAAV5-hIL-10/hGDNF.

Through the results described above, the present inventors could confirm that when the gene therapeutic agent (rAAV5-hIL-10/hGDNF) of the present invention is administered, it is possible to not only prevent but also treat the hypokinesia symptoms of a degenerative brain disease and the preventive and therapeutic effects are maintained for a long period of time.

### Example 2. Confirmation of degenerative brain disease preventive effect when rAAVS-hIL-10/hGDNF and rAAV5-hGAD65 are simultaneously administered

### 2-1. Confirmation of protective effects of rAAVS-hIL-10/hGDNF and rAAV5-hGAD65 on dopaminergic neurons in animal model

In order to confirm the effect of rAAVS-hIL-10/hGDNF and rAAV5-hGAD65 (simultaneously administered), which are one of the gene therapeutic agents of the present invention, on the suppression of dopaminergic neuronal cell death, after pretreating the mice with rAAV5-hIL-10/hGDNF and rAAV5-hGAD65, 2 weeks later, a Parkinson's disease animal model was produced by administering 6-OHDA. The procedure was performed in the same manner as in Example 2-2 to confirm the protective effect on dopaminergic neurons in the striatum (ST) in which the axons of the substantia nigra pars compacta (SNpc) and SNpc cells extend.

As a result, as shown in FIG. 3A, the case where a group was pretreated with rAAV5-hIL-10/hGDNF and rAAV5-hGAD65 showed a significantly lower level of dopaminergic neuronal cell death in the striatum compared to the case where a group was not pretreated with rAAV5-hIL-10/hGDNF and rAAV5-hGAD65, and, as shown in FIG. 3B, it could also be confirmed that the group pretreated with rAAV5-hIL-10/hGDNF and rAAV5-hGAD65 showed a significantly lower level of dopaminergic neuronal cell death in the SNpc compared to the group treated with 6-OHDA alone.

### 2-2. Confirmation of amelioration effects of rAAVS-hIL-10/hGDNF and rAAV5-hGAD65 on hypokinesia symptoms in degenerative brain disease animal model

Two weeks before producing a mouse model by the method of Example 2-1 above, the mice were pretreated with rAAV5-hIL-10/hGDNF and rAAV5-hGAD65, and a rotation test (apomorphine induced rotation) and a rota-rod test were performed on the degenerative brain disease mouse model produced by the method described above. Specific experimental methods are shown in the following Table 2.

**[Table 2]**

| **Group** | **Animals** | **Injection materials** | |
|---|---|---|---|
| | | **Test article** | **6-OHDA lesion surgery** |
| **Saline+0.1%AA** | C57BL/6, male (9 weeks) | SN: 2 µL Saline, | ST: 2 µL 0.1% ascorbic acid (AA) |
| | | ST: 2 µL Saline | |
| **Saline+6-OHDA** | | SN: 2 µL Saline, | ST: 18 µg 6-OHDA /2 µL 0.1%AA |
| | | ST: 2 µL Saline | |
| **rAAV5-hIL-10/hGDNF** + **rAAV5-hGAD65 +6-OHDA** | | SN: rAAV5-hGAD65 5×10¹⁰ VG/2 µL, | |
| | | ST: rAAV5-hGDNF/hIL-10 5×10¹⁰ VG/2 µL | |

### ① Rotation test results

The rotation test was performed in the same manner as in Example 1-3, and as a result, it could be confirmed that, when the mice administered the gene therapeutic agent of the present invention was compared with the control mice administered 6-OHDA alone on week 4 of model production, an effective level of amelioration of motor function decline was shown (FIG. 4A), and for the amelioration effect, as shown in FIG. 4B, it could be confirmed that, after a degenerative brain disease mouse model was produced by administering 6-OHDA, an effective level of hypokinesia amelioration effect was shown compared to the control, even after 10 weeks had passed (FIG. 4B).

Through the results described above, the present inventors could confirm that when the gene therapeutic agent (simultaneous administration of rAAV5-hIL-10/hGDNF and rAAV5-hGAD65) of the present invention is administered, it is possible to prevent the hypokinesia symptoms of the degenerative brain disease and the preventive effect is maintained for a long period of time.

### ② Rota-rod test results

In the rota-rod test, the mice were placed on a rota-rod behavioral test apparatus cylinder and then were given time to adapt to the apparatus by rotating the disk clockwise at a speed of 5 RPM for 2 minutes. The time the mouse walked on the cylinder was measured while rotating the cylinder at various RPMs, the experiment was performed in triplicate per individual, and the average of the three measurement values was shown as the representative value of the individual.

As a result, as shown in FIG. 4C, when 2 weeks had elapsed after administration of 6-OHDA, regarding the decline in motor function, which was confirmed to be significantly reduced, it could be observed that, for the case of pretreatment with rAAV5-hIL-10/hGDNF and rAAV5-hGAD65 of the present invention, it was shown at a level similar to that of the same treatment control group (sham control) which was simply administered 0.1% ascorbic acid.

### Example 3. Confirmation of effect of co-administration of hIL-10 and hGDNF protein cultures produced by plasmid or simultaneous administration of protein cultures of hIL-10, hGDNF and hGAD65 on suppression of neuronal cell death caused by oxidative stress

In order to confirm the effect of suppressing neuronal cell death caused by oxidative stress upon co-administration of hIL-10 and hGDNF protein cultures or simultaneous administration of protein cultures of hIL-10, hGDNF and hGAD65, a medium containing hIL-10 and hGDNF proteins or hIL-10, hGDNF and hGAD65 proteins, produced after transfection with hIL-10, hGDNF and hGAD65, was pretreated, and the mock and control were treated with a p-Vex medium and then treated with H₂O₂ or 6-OHDA, which induce neuronal cell death, to confirm cell viability.

As a result, as can be seen in FIGS. 5A and 5B, it was confirmed that both co-administration of hIL-10 and hGDNF protein cultures or simultaneous administration of protein cultures of hIL-10, hGDNF and hGAD65 suppress neuronal cell death.

Through these results, it could be seen that not only the genes of hIL-10 and hGDNF, but also their respective proteins have preventive or therapeutic effects on degenerative brain diseases, and that they are effective against various degenerative brain diseases including Parkinson's disease because they have a neuroprotective effect regardless of the cause of neuronal cell death.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

## Claims

1. A pharmaceutical composition for preventing or treating a degenerative brain disease, comprising two or more selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

2. The pharmaceutical composition of claim 1, comprising the IL-10 protein or the gene encoding the same and the GDNF protein or the gene encoding the same.

3. The pharmaceutical composition of claim 2, further comprising the GAD protein or the gene encoding the same.

4. The pharmaceutical composition of claim 1, wherein the gene is comprised in a vector.

5. The pharmaceutical composition of claim 4, wherein the vector is a viral or non-viral vector.

6. The pharmaceutical composition of claim 5, wherein the viral vector is one or more selected from the group consisting of adenoviruses, adeno-associated viruses (AAV), herpes simplex virus, lentiviruses, retroviruses, cytomegalovirus, baculoviruses, and poxviruses.

7. The pharmaceutical composition of claim 5, wherein the non-viral vector is one or more selected from the group consisting of plasmids, liposomes, cationic polymers, micelles, emulsions, and solid lipid nanoparticles.

8. The pharmaceutical composition of claim 1, wherein the IL-10 protein is an amino acid sequence represented by SEQ ID NO: 1.

9. The pharmaceutical composition of claim 1, wherein the IL-10 encoding gene is a base sequence represented by SEQ ID NO: 2 or SEQ ID NO: 3.

10. The pharmaceutical composition of claim 1, wherein the GDNF protein is an amino acid sequence represented by SEQ ID NO: 4.

11. The pharmaceutical composition of claim 1, wherein the GDNF protein encoding gene is a base sequence represented by SEQ ID NO: 5 or SEQ ID NO: 6.

12. The pharmaceutical composition of claim 1, wherein the GAD is GAD 65 or GAD 67.

13. The pharmaceutical composition of claim 1, wherein the GAD protein is an amino acid sequence represented by SEQ ID NO: 7 or SEQ ID NO: 9.

14. The pharmaceutical composition of claim 1, wherein the GAD protein encoding gene is a base sequence represented by SEQ ID NO: 8 or SEQ ID NO: 10.

15. The pharmaceutical composition of claim 1, wherein the degenerative brain disease is one or more selected from the group consisting of Parkinson's disease, dementia, Alzheimer's disease, Huntington's disease, frontotemporal lobar degeneration, dementia with Lewy bodies, vascular dementia, corticobasal degeneration, multiple system atrophy, progressive supranuclear palsy, primary lateral sclerosis, spinal muscular atrophy, stroke, cerebral infarction, head trauma, spinal cord injury, cerebral arteriosclerosis, Lou Gehrig's disease, and multiple sclerosis disease.

16. The pharmaceutical composition of claim 1, further comprising a physiologically acceptable carrier.

17. The pharmaceutical composition of claim 1, wherein the pharmaceutical composition is an injection.

18. The pharmaceutical composition of claim 1, wherein the pharmaceutical composition has an effect of ameliorating motor abnormalities caused by neuronal cell death.

19. The pharmaceutical composition of claim 18, wherein the neurons are motor neurons, sensory neurons or interneurons.

20. The pharmaceutical composition of claim 1, wherein the pharmaceutical composition has an effect of improving cognitive ability and memory.

21. A health functional food for preventing or ameliorating a degenerative brain disease, comprising two or more selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same.

22. A method for preventing or treating a degenerative brain disease, the method comprising administering two or more selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same to an individual in need thereof.

23. A use of two or more selected from the group consisting of an interleukin 10 (IL-10) protein or a gene encoding the same, a glial cell line-derived neurotrophic factor (GDNF) protein or a gene encoding the same, and a glutamate decarboxylase (GAD) protein or a gene encoding the same for preparing a preventive or therapeutic agent for degenerative brain diseases.
